# EUROPÄISCHE PATENTSCHRIFT

(11) **EP 1 296 609 B1**
(45) Veröffentlichungstag und Bekanntmachung des Hinweises auf die Patenterteilung: **12.01.2005**
(21) Anmeldenummer: 01947424.6
(22) Anmeldetag: 28.06.2001
(51) Int. Cl.: A61B 19/00

(54) **MEDIZINISCHE VORRICHTUNG FÜR STEREOTAXIE UND PATIENTENPOSITIONIERUNG**
MEDICAL DEVICE FOR STEREOTAXIS AND PATIENT POSITIONING
DISPOSITIF MEDICAL POUR LA STEREOTAXIE ET LE POSITIONNEMENT D'UN PATIENT

(30) Priorität: 01.07.2000 DE 10032203
(43) Veröffentlichungstag der Anmeldung: 02.04.2003
(73) Patentinhaber: Deutsches Krebsforschungszentrum Stiftung des öffentlichen Rechts, 69120 Heidelberg (DE)
(72) Erfinder: SCHLEGEL, Wolfgang, 69118 Heidelberg (DE); PASTYR, Otto, 69181 Leimen (DE); ECHNER, Gernot, 69257 Wiesenbach (DE); STURM, Volker, 69168 Wiesloch-Schatthausen (DE)
(74) Vertreter: Weber, Walter, Dipl.-Ing.(FH)
(86) Internationale Anmeldenummer: PCT/EP2001/007440
(87) Internationale Veröffentlichungsnummer: WO 2002/003878

(56) Entgegenhaltungen:
- WO-A-00/33755
- WO-A-01/76499
- WO-A-99/37220
- US-A- 5 480 114

## Beschreibung

Die Erfindung betrifft eine medizinische Vorrichtung mit einer Mechanik zur Positionierung einer Einrichtung mittels eines Armes, welcher aus mindestens drei Gliedern besteht, wobei die Mechanik mindestens fünf Stellachsen aufweist, welchen Feineinstell- und Arretiervorrichtungen zugeordnet sind.

Derartige medizinische Vorrichtungen dienen in der Stereotaxie dem Einsatz bei verschiedenen diagnostischen und therapeutischen Zwecken wie der Gewebeprobenentnahme, der Evakuierung von Hämatomen, der Punktion von Zysten, dem gezielten Lasereinsatz oder Implantationen für viele interventionelle radiologische Anwendungen mittels Computertomographie, Magnetresonanztomographie, Positronen-Emissionstomographie usw., zum Beispiel zur Einbringung radioaktiver Implantate zur Krebsbehandlung. Dabei wird anhand einer Diagnose mit Hilfe eines Lokalisationssystems, beispielsweise einem der vorgenannten, der Zielpunkt und die Sondenrichtung bestimmt. Diese Bestimmung ist eine ärztliche Festlegung, wobei diese oftmals mit Hilfe der Berechnung durch ein Planungsprogramm vorgenommen wird. Beispielsweise kann der Zielpunkt eine Gewebeentnahme an einem Tumor sein und die Sondenrichtung zu diesem Zielpunkt wird derart bestimmt, daß der Vorstoß zum Zielpunkt möglichst ohne nachteilige Folgen bleibt, also empfindliche Organe umgeht. Derartige Eingriffe erfordern eine derart hohe Präzision und Sicherheit, daß sie in der Regel mittels Positionierungs- und Leitsystemen für Sonden durchgeführt werden, welche die Einbringrichtung und oftmals auch den Zielpunkt zur Erzielung der notwendigen Exaktheit und Sicherheit mechanisch vorgeben.

Eine Vorrichtung der eingangs genannten Art für die Stereotaxie ist aus der US 6, 035, 228 bekannt. Diese Stereotaxievorrichtung ist an einer bildgebenden Einrichtung auf einer quer zum Patienten verlaufenden Schiene angebracht und kann daher mittels der Daten dieser bildgebenden Einrichtung Instrumente zu einem Zielpunkt führen. Als bildgebende Einrichtung wird dabei eine röhrenartige Röntgenvorrichtung vorgeschlagen, in welche der Patient eingeschoben wird und die daher, den Patienten umgebend, eine allseitige Bilderfassung ermöglicht. Diese Stereotaxievorrichtung ist bereits für den oben genannten Einsatz nur bedingt verwendbar, da es durch die feste nur auf der Schiene verschiebbare Zuordnung zum bildgebenden Gerät nicht uneingeschränkt jeden Zielpunkt des menschlichen Körpers in jeder Richtung erreichen kann. Da der Arm dieser Vorrichtung zwei aufeinanderfolgende, gleich ausgerichtete Drehachsen aufweist, sind die Freiheitsgerade der Bewegungsmöglichkeiten beschränkt, was einer universellen Einsetzbarkeit entgegensteht. Die Vorrichtung ist zum Einsatz von chirurgischen Instrumenten auf der Grundlage der Daten des bildgebenden Geräts im wesentlichen zur Gewebeprobeentnahme konzipiert und bestimmt. Durch die feste Verbindung zu dem portalartig den Patienten umgebenden bildgebenden Gerät kann die bekannte Stereotaxievorrichtung nicht jeden beliebigen Standort zum Patienten einnehmen. Daher ist sie nicht für Operationen, zumindest nicht für größere Operationen einsetzbar, da das portalartige Gerät den allseitigen Zugang zum Patienten versperrt.

Darüber hinaus ist ein wichtiges weiteres Einsatzbedürfnis nicht Gegenstand der vorbekannten Vorrichtung. Außer dem Anfahren von Zielpunkten zur Behandlung oder Diagnose besteht das Bedürfnis, die Position eines Patienten zu vermessen, beispielsweise, um bei immer wiederkehrenden Behandlungen den jedesmaligen Einsatz eines bildgebenden Geräts zu vermeiden. Dadurch wird nicht nur der Aufwand verringert, es wird auch eine Schädigung durch oft wiederholten Einsatz der Bildgebung, z. B. durch ein Röntgengerät, vermieden.

Aus der WO 00/33755 ist ein chirurgischer Instrumentenhalter mit einem beweg- und justierbaren Arm bekannt. Dabei ist ein Ständer mit einer Höhenverstellung für den Arm vorgesehen. Der Arm weist mehrere Glieder auf, wobei jedoch die ersten drei Stellachsen alle senkrecht ausgerichtet sind. Dadurch sind die Freiheitsgerade der Verstellung sehr eingeschränkt und die Einsetzbarkeit ist dadurch sehr beschränkt, im wesentlichen auf das Halten von Instrumenten.

Aus der WO 99/37220 ist ein orthopädisches Gerät für Amputationen mit Armen für das Halten von Gliedmaßen und Werkzeugen bekannt. Auch diese Arme weisen mehrere aneinandergereihte Gelenke mit gleicher Drehachsenausrichtung auf, was ebenfalls eingeschränkte Stellmöglichkeiten und beschränkte Einsatzmöglichkeiten zur Folge hat.

Aus der WO 01/76499A2 ist weiterhin ein Stand der Technik gem. Art. 54 Abs. 3 EPÜ bekannt, der eine Mechanik zur Positionierung eines mehrgliedrigen Arms aufweist, bei dem mit einer einzigen Arretiervorrichtung eine einmal gefundene Einstellung des Arms durch eine Betätigung der Arretiervorrichtung festlegbar ist. Eine Feineinstellung und Arretierung einzelner Stellachsen ist bei dieser Mechanik jedoch nicht möglich. Aus diesem Grund kann nur eine gefundene Einstellung für die Dauer der Aufrechterhaltung der Arretierstellung erhalten werden, wird die Arretierung gelöst, ist die gefundene Einstellung verloren. Es ist auch weder die Reproduktion einer Einstellung mit Hilfe von Daten, noch die Vermessung der Position eines Patienten möglich. Der Einsatzbereich ist daher wie erwähnt sehr beschränkt.

Der Erfindung liegt daher die Aufgabe zugrunde, eine Vorrichtung der eingangs genannten Art derart auszugestalten, daß sie universell einsetzbar ist, insbesondere als beliebig plazierbare und weitere ärztliche Maßnahmen wenig behindernde Vorrichtung für die Stereotaxie sowie als ebenso beliebig plazierbare Lagebestimmungsvorrichtung zur Ermittlung der Lage eines Patienten.

Die Aufgabe wird durch eine medizinische Vorrichtung gemäß Anspruch 1 gelöst.

Mechaniken, die auf einem vergleichbaren Konstruktionsprinzip beruhen, wie die erfindungsgemäße Vorrichtung, sind zwar als Handhabungs- und Meßroboter bekannt, diese sind jedoch in der bestehenden Form für medizinische Eingriffe nur geeignet, wenn ein großer technischer Aufwand vorgenommen wird, damit ein derartiger Roboter den Sicherheitsanforderungen des Medizinproduktegesetzes entspricht. Durch einen derartigen technischen Aufwand wäre ein solcher Roboter für den allgemeinen Einsatz zu teuer. Andererseits beruht die Konzeption eines derartigen Roboters auf anderen, bezüglich Sicherheitsanforderungen geringeren aber bezüglich der Bewegungskoordination wesentlich höheren Anforderungen. Derartige Roboter müssen alle Arten von Bewegungen ausführen, um beispielsweise Montagearbeiten zu erbringen. Der vorlie gende medizinische Einsatzbereich beschränkt sich jedoch darauf, eine Einrichtung bezüglich einer Bewegungsrichtung und bezüglich ihrer Ausrichtung auf einen Zielpunkt am oder im Körper zu fixieren, um diesen aus einer Position außerhalb des Körpers durch eine geradlinige Verschiebung exakt und sicher zu erreichen, wobei selbstverständlich der Patient oder das entsprechende Körperteil des Patienten fixiert werden muß. Solche Fixierungen sind beispielsweise aus der Strahlentherapie bekannt. Aus den vorgenannten Gründen kann bei der erfindungsgemäßen Vorrichtung auf eine äußerst aufwendige Bewegungssteuerung im dreidimensionalen Raum verzichtet werden. Es reicht, wenn alle Stellachsen nacheinander eingestellt werden, damit der Arzt nach erfolgter Positionierung einer Einrichtung, beispielsweise einer Sonde, und Überprüfung dieser Positionierung diese in der eingestellten Zielrichtung zu einem vorbestimmten Zielpunkt in den Körper einführen und die erforderlichen Tätigkeiten am Zielpunkt vornehmen kann.

In entsprechender Weise reicht es, wenn eine zu erfassende Position eines Zielpunktes aus einer Richtung angefahren wird. Auch hier ist die Erreichung des Zielpunktes, z. B. einer Markierung oder eines Eichpunktes, erforderlich - der Weg dorthin ist, wenn der Zielpunkt auf dem Körper oder außerhalb desselben angeordnet ist, in der Regel nicht von Bedeutung. Es ist also auch hier kein koordinierter Bewegungsablauf im dreidimensionalen Raum zu steuern.

Wenn auch die erfindungsgemäße Vorrichtung mit Antrieben versehen werden, sowie computergesteuert betrieben werden kann, so beschränkt sich die Betätigung immer noch auf die Vornahme einer Reihe von Einstellungen, die nacheinander beziehungsweise unabhängig voneinander vorgenommen werden können, und erfordert keinen gleichzeitigen koordinierten Bewegungsablauf in mehreren Gelenken. Eine entsprechende Berechnung der Einstellungen kann vor Vornahme derselben erfolgen und sie ist wesentlich unaufwendiger als die Koordinierung mehrerer gleichzeitig vorzunehmender Stellbewegungen.

Ein ganz wesentlicher Aspekt ergibt sich bezüglich der Sicherheit: Die Einstellungen können ohne jegliche Gefährdung eines Patienten durch motorangetriebene Komponenten vorgenommen und gründlich überprüft werden. Die Stellbewegung bei der Behandlung beschränkt sich dann darauf, auf dem eingestellten und überprüften Stellweg mit einer Einrichtung, wie einer Sonde zum Zielpunkt zu gelangen. Die den menschlichen Körper tangierende Stellbewegung ist also eindimensional und nicht wie die von einem Roboter geforderten Bewegungen dreidimensional. Sie kann daher ohne allzu großen technischen Aufwand mit hohem Sicherheitsstandart überprüft und durchgeführt werden. Die Einstellungen können daher mit einer sehr hohen Präzision erfolgen und benötigen keinen kostspieligen Steuerungsaufwand. Der Erreichung und Fixierung der vorgenannten Position der Sonde dienen erfindungsgemäß die Zuordnung von Feineinstell- und Arretiervorrichtungen zu den Stellachsen.

Durch die Ausgestaltung der Vorrichtung mit einem beliebig im Bereich des Patienten plazierbaren Ständerteil, das leicht beweglich ist, wird die erfindungsgemäße medizinische Vorrichtung nicht nur in universeller Weise für die verschiedensten Eingriffe und Untersuchungen in der Stereotaxie sondern auch noch in weiteren Bereichen, wie der Patientenpositionierung einsetzbar. Bei letzterem wird nicht wie bei der Stereotaxie ein vorbestimmter Zielpunkt angesteuert, sondern ein oder mehrere Zielpunkte am Patienten, z. B. Markierungen angefahren und daraus die Lage des Patienten bestimmt. Aufgrund der Lagebestimmung kann entweder die Positionierung des Patienten geändert werden oder vorzugsweise wird aufgrund der eingenommenen Lage berechnet, wo sich der zu behandelnde oder zu untersuchende Raum befindet. Danach kann die Behandlung vorgenommen werden, die beliebig sein kann, als Bestrahlung oder als Behandlung oder Untersuchung, die ebenfalls mit der erfindungsgemäßen Vorrichtung durchführbar ist. Ein besonderer Vorteil der Erfindung besteht darin, daß die Vorrichtung sowohl zur Patientenpositionierung als auch zur Behandlung eingesetzt werden kann. Ersteres dient dazu, die Lage des Patienten zu erfassen, zu korrigieren oder rechnerisch zu berücksichtigen und zu verifizieren. Danach kann das der Behandlung oder Untersuchung dienende Gerät am Arm der Vorrichtung befestigt und der Eingriff durchgeführt werden.

Auf diese Weise erspart diese Verwendung der erfindungsgemäßen Vorrichtung - gegebenenfalls mit den entsprechenden Ausgestaltungen zu diesem Zweck - bei sich wiederholenden Behandlungen oder Untersuchungen, die jedesmal erneut erforderliche Bildgebung, wie dies beim Gegenstand der US 6, 035, 228 erforderlich ist. Der besondere Vorteil des erfindungsgemäßen Gegenstandes besteht aber darin, daß er in hohem Maße universell einsetzbar ist, insbesondere für aufeinanderfolgende Arbeitsschritte wie Positionierung, gegebenenfalls weitere Untersuchung und Behandlung.

Die Erfindung hat den Vorteil, daß sie für Behandlung, Diagnose und Positionsbestimmung im Bereich des ganzen Körpers einsetzbar ist, wobei eine Einrichtung in nahezu jeder Richtung jeden beliebigen Punkt erreichen kann. Bei derartigen Einrichtungen kann es sich um alle Einrichtungen für Eingriffe, Diagnose und Positionsbestimmungen handeln. Es wird auf die eingangs genannten Anwendungen verwiesen, aber auch Positionsbestimmungen mittels Vorrichtungen, wie Taster und vieles weitere ist möglich. Für Eingriffe oder Untersuchungen hat dies den Vorteil, daß Zielpunkte aus allen Richtungen angefahren werden können, es kann also bei stereotaktischen Eingriffen die für den Patienten schonendste Einbringrichtung gewählt werden. Zielpunkte können je nach Einsatzbereich der Vorrichtung im Körper, auf dem Körper oder an einem Teil sein, das mit dem Körper in fester Verbindung steht. Da jeder beliebige Standort für die Mechanik möglich ist, kann eine Auswahl des Standortes derart getroffen werden, daß unter Beibehaltung einer optimalen Einbringrichtung die Mechanik derart positioniert ist, daß sie bei weiteren Behandlungsmaßnahmen nicht im Weg ist. Die Mechanik ist mit hoher Präzision einstellbar und es können Sonden oder sonstige Einrichtungen aller Art eingesetzt werden, auch solche, die der Positionsbestimmung zur Patientenpositionierung dienen - eine Einsatzmöglichkeit, auf die weiter unten noch eingegangen wird.

Für das Ständerteil ist wesentlich, daß es leicht an alle möglichen Standorte gebracht werden und an diesen jedoch unverrückbar positioniert werden kann. Ein Standort muß dabei nicht auf dem Boden sein, auch Befestigungen an anderen Gegenständen oder an der Decke sind möglich - wesentlich ist, daß die Positionierung in hohem Maß variabel ist. Für die Unverrückbarkeit kann vorgesehen sein, daß das Ständerteil eine Einrichtung zur Unterbindung einer ungewollten Standortveränderung aufweist. So kann es auf dem Boden im Bereich des Patienten fixierbar sein. Dann ist es zweckmäßig, wenn das Ständerteil so klein ausgestaltet ist, daß es noch genügend Standsicherheit gewährleistet, ansonsten jedoch keinen Raum beansprucht, der für ärztliche Maßnahmen oder weitere Geräte zur Verfügung stehen muß. Eine leichte Positionsveränderung trotz hoher Standsicherheit kann beispielsweise dadurch erzielt werden, daß das Ständerteil Rollen und eine Einrichtung zur Unterbindung der Rollenbewegung aufweist, beispielsweise können festlegbare Bremsen vorgesehen sein. Es sind jedoch auch andere Einrichtungen zur Fixierung der Vorrichtung denkbar, wie ausfahrbare Ständer, einfahrbare Rollen Magnethalterungen oder ähnliches.

Eine weitere Möglichkeit der Fixierung an einem Standort besteht darin, daß das Ständerteil mit einem Teil verbindbar ist, das einen festen Bezug zum Patienten aufweist. Beispielsweise kann das Ständerteil mit einer Fixiervorrichtung verbindbar sein. Wird die Vorrichtung für Eingriffe im Kopfbereich relativ klein ausgebildet, so kann das Ständerteil an einem Fixierungsring für den Kopf angebracht werden. Die Anbringung an allen Arten von Fixierungsvorrichtungen ist jedoch denkbar oder eine Verbindung mit dem Patiententisch, insbesondere, wenn dieser mit Fixiervorrichtungen ausgestattet ist. Auch hier sollten die Positionierungsmöglichkeiten möglichst variabel sein.

Ein wesentlicher Vorteil der Erfindung im Vergleich zum aus der US 6, 035, 228 bekannten Stand der Technik besteht darin, daß die Vorrichtung nicht mit dem Tomographiegerät verbunden ist, sondern zu dem Patienten in eine feste Verbindung gebracht ist, da gerade diese Tatsache den fortwährenden Einsatz des Tomographiegeräts zur ständigen Positionserfassung erspart. Dies ist in Anbetracht der schädlichen Röntgenstrahlen besonders wichtig und ermöglicht zusätzlich Maßnahmen, bei denen ein Tomographiegerät im Weg wäre. Besonders vorteilhaft ist die feste Verbindung zum Patienten, die nicht über den Boden, sondern über ein Teil erfolgt, mit dem dieser fixiert ist, wie ein Kopfhalterungsring oder eine Fixicrmaßnahme an der Patientenliege. Dann ist es möglich, daß der Patient mit der Vorrichtung zwischen dem Operationsbereich und einer bildgebenden Einrichtung (Röntgengerät, Magnetresonanztomographiegerät) hin und her verbracht wird, was eine Unterbrechung eines Eingriffs und eine Fortsetzung nach einer Verifikation ermöglicht. So kann beispielsweise festgestellt werden, ob eine gesetzte Biopsienadel exakt in Richtung des Zielpunktes ausgerichtet ist oder einer Korrektur bedarf. Dadurch kann eine bisher nicht mögliche Präzision erreicht werden und zwar auch bei Eingriffen, die nicht durch ständige Bildgebung überwacht werden können oder sollen.

Zweckmäßigerweise wird bei der Mechanik der erfindungsgemäßen Vorrichtung eine weitere sechste Stellachse vorgesehen, der ebenfalls eine Feineinstell- und Arretiervorrichtung zugeordnet ist. Auf diese Weise wird die Vorrichtung noch universeller einsetzbar und für die Erreichbarkeit beliebiger Punkte aus jeder beliebigen Richtung gibt es keine Beschränkungen mehr.

Vorzugsweise wird das vorderste Glied mit einem Halter zur Aufnahme verschiedenster Einrichtungen ausgestattet. Dadurch ist der Einsatz zu allen denkbaren diagnostischen und therapeutischen Zwecken sowie zur Positionsbestimmung möglich. Dies kann der geradlinigen Einbringung einer Sonde dienen, da dafür eine exakt geradlinige Bewegung erforderlich ist. Dabei kann auch vorgesehen sein, daß das vordere Glied des Arms für die geradlinige Einbringung einer Einrichtung eine in Richtung auf einen Zielpunkt richtbare Führung aufweist. Beispielsweise kann das vordere Glied teleskopartig ausgebildet sein. Wird ein Halter mit einer solchen Führung ausgestattet, so kann diese parallel oder in einem Winkel zum vorderen Glied verlaufen. Letzteres hat den Vorteil, daß die Vorrichtung nicht im Weg ist, wenn der Arzt möglichst nahe an den Behandlungsbereich herankommen will. Für die geradlinige Bewegung einer Einrichtung gibt es jedoch noch weitere Möglichkeiten, beispielsweise kann am Arm eine weitere Stellachse vorgesehen sein, die zu zwei Stellachsen, deren Glieder in derselben oder parallelen Ebenen bewegbar sind, eine derartige Kopplung aufweist, daß die Stellachsen gegenläufige Winkelverstellungen in sich gegenseitig aufhebender Weise vollziehen und gleichzeitig eine Korrektur zum Verbleib der Lage der Einrichtung in Richtung auf einen Zielpunkt durchführen können. Diese Mechanik verhindert somit ebenso eine Winkelverstellung als auch eine Parallelverschiebung der Sonde, während diese auf einen Zielpunkt zubewegt wird. Letztlich ist jede Ausgestaltung möglich, die eine geradlinige Einbringung beispielsweise einer Sonde mit großer Exaktheit und Sicherheit garantiert. In entsprechender Weise auch jede Ausgestaltung, bei der sich mittels der Positionierung und der Winkelstellungen des Arms die Lage eines der Positionserfassung und Korrektur dienenden Zielpunktes ermitteln läßt.

Die für die Positionierungen erforderlichen Winkelstellungen für die Stellachsen können auf beliebige Art und Weise ermittelt werden. Ebenso kann die zur Positionserfassung eines Zielpunktes erforderliche Erfassung der Ständerpositionierung und der Winkelstellungen der Glieder auf verschiedene Weise erfolgen. Diese lassen sich z. B. durch eine digitale Kamera mit entsprechender Bildverarbeitung von außen erfassen oder die Stellachsen können mit Winkelmessern ausgestattet sein. Weiterhin kann eine Einrichtung zur Bestimmung der Koordinaten beliebiger Standorte des Ständers auf dem Boden, an der Decke oder eines zur Anbringung ausgestalteten Standorts in Bezug auf ein Teil, das in einer festen Beziehung zum Patienten steht, vorgesehen sein. Dabei kann es sich um eine Erfassung des Standortes in Form einer Einrichtung im Boden oder an der Decke, einer digitalen Kamera oder in der weiter unten noch erläuterten Weise handeln.

Aus Gründen der Zweckmäßigkeit und der Zeitökonomie wird vorgeschlagen, daß die Vorrichtung zusätzlich mit einem Rechner verbindbar ist und ein Programm für den Rechner aufweist, das über die Winkelstellungen der Stallachsen einen frei wählbaren Zielpunkt und einen frei wählbaren Standort des Ständers derart zueinander in Bezug setzt, daß einer davon aufgrund der Koordinaten des anderen errechenbar ist. Dies eröffnet einen universellen Einsatz einmal dahingehend, daß das Programm für Standorte des Ständers die Winkelstellungen der Stellachsen zur Erreichung eines Zielpunktes errechnet. Die ermittelten Werte können dann beispielsweise an entsprechenden Einstellskalen der Feineinstellungen der Stellachsen eingestellt werden. Es kann jedoch auch vorgesehen sein, daß die Stellachsen mit Winkelmessern ausgestattet sind, wodurch sich eine größere Anzeigegenauigkeit und damit eine größere Einstellgenauigkeit der Winkel erzielen läßt.

Der andere Einsatz dient dem Zweck der Bestimmung der Position des Patienten. Dazu kann vorgesehen sein, daß die Vorrichtung ein Programm für einen Rechner aufweist, das aufgrund des Standorts des Ständers und der Winkelstellungen der Stellachsen die Position eines durch eine Sonde angefahrenen Zielpunktes errechnet. Mittels der auf diese Weise gewonnenen Daten kann der Patient für eine Behandlung oder Untersuchung positioniert werden, wobei vorzugsweise eine "elektronische Positionierung" erfolgt, indem aufgrund der Position des Patienten die Daten für die vorzunehmende Behandlung, z. B. Bestrahlung oder Stereotaxie in Form einer Koordinatentransformation umgerechnet werden.

Zur Bestimmung der Positionen des Ständers ist es auch möglich, daß der Arm mit einer Positionsbestimmungsvorrichtung ausstattbar oder ausgestattet ist. Beispielsweise kann am vorderen Ende des Arms ein Taster angeordnet, zum Beispiel in dem Sondenhalter befestigt werden. Mittels der Positionsbestimmungsvorrichtung ist es möglich, durch Anfahren von Bezugspunkten, beispielsweise eines Lokalisationssystems, zuerst die Position des Ständers zum Zielpunkt zu ermitteln und dann die für die gewünschte Sondenpositionierung erforderlichen Winkelstellungen der Stellachsen bezogen auf den gewählten Standort zu errechnen und einzustellen, um einen Zielpunkt anzufahren. In derselben Weise können auch Zielpunkte am Patienten angefahren werden, um dessen Lage zu bestimmen, zu überprüfen oder zu korrigieren.

Eine Weiterbildung sieht vor, daß die Vorrichtung ein Programm für einen Rechner aufweist, das mittels dreier als Zielpunkte definierter Eichpunkte die Lage beliebig gewählter Standorte des Ständers bestimmt. Auf diese Weise läßt sich in analoger Art wie die Zielpunktbestimmung aufgrund eines definierten Standorts des Ständers oder umgekehrt über das Anfahren von Eichpunkten der Standort des Ständers bestimmen. Weisen die Eichpunkte einen definierten Bezug zum Patienten auf, so lassen sich die Koordinaten von Ständer und Zielpunkt derart einander zuordnen, daß für jeden Standort des Ständers eine sichere Erreichung eines Zielpunktes möglich ist: Gegebenenfalls ist der Standort zu ändern, wenn dies zur Beschreitung eines möglichen Einbringweges erforderlich ist. Es ist auch möglich, Lagefehler des Patienten zu berücksichtigen, indem die Koordinatensysteme unter Eliminierung der Fehler einander zugeordnet werden.

Zur Positionsbestimmung der Position des Patienten ist das Programm zweckmäßigerweise derart ausgebildet, daß es nach der Bestimmung des Standortes des Ständers mittels der Eichpunkte aus den Winkelstellungen der Stellachsen die Lage mindestens eines angefahrenen Zielpunktes überprüft. Dazu können mehrere als Zielpunkte dienende Markierungen am Patienten oder Markierungen mit festem Bezug zum Patienten zur Bestimmung und/oder Überprüfung von dessen Lage dienen. Die Markierungen können auf der Haut des Patienten aufgebracht, an Schalen zur Aufnahme von Körperteilen oder einer anderen Fixiervorrichtung, wie einem Fixierungsring für den Kopf oder an einem Gebißstück, angebracht sein. Die Möglichkeit der beliebigen Anordnung des Ständers im Bereich der Patienten ist auch für die vorgenannte Positionsbestimmung von Vorteil, da Markierungen an beliebigen Stellen erfaßt werden können.

Ein Programm für Untersuchungen und Eingriffe ist zweckmäßigerweise derart ausgebildet, daß es den Einbringweg bis zur Erreichung eines im Körper eines Patienten liegenden Zielpunktes ermittelt, um auch hier eine größere Genauigkeit zu erzielen. Dann ist es zweckmäßig, für den Einbringweg eine Meßvorrichtung vorzusehen, die mit einer entsprechend genauen Anzeige ausgestattet ist. Für alle Brauchbarkeiten ist es zweckmäßig, wenn die Vorrichtung mit einem Rechner ausgestattet ist, welcher zur Bestimmung der Lage mindestens eines Zielpunktes zum Standort des Ständers oder umgekehrt mit einem Programm geladen ist.

Die Vorrichtung besteht mindestens aus der genannten Mechanik und zweckmäßigerweise aus einem der vorgenannten Programme. Es kann dabei ein handelsüblicher Rechner herangezogen werden oder es ist möglich, daß die Vorrichtung mit einem Rechner ausgestattet ist, welcher mit einem der vorgenannten Programme geladen und gegebenenfalls für die vorliegende Anwendung modifiziert ist. Es ist auch möglich, einen Spezialrechner für den genannten Zweck vorzusehen.

Für eine schnelle Arbeitsweise wird vorgeschlagen, daß die Winkelmesser und die Positionsbestimmungsvorrichtung mit einem Rechner verbunden sind, wobei dieser die Daten des Standortes des Ständers, der Einstellung der Mechanik und die Daten eines Zielpunktes ermittelt. Dies kann der Erreichung eines vorgegebenen Zielpunktes oder der Bestimmung der Lage eines angefahrenen Zielpunktes dienen. Im erstgenannten Fall kann das Programm derart gestaltet sein, daß es die Daten unmittelbar umsetzt oder daß es einer Bedienperson die vorzunehmenden Winkeleinstellungen mitteilt. Die Mitteilung kann erfolgen, indem das Programm derart ausgebildet ist, daß ein Rechner die zur Erreichung des Zielpunktes erforderlichen Einstellungen oder auch sonstige wichtige Daten an einer Anzeigeeinrichtung, in der Regel auf dem Bildschirm, anzeigt. Zweckmäßigerweise werden auch die Einstellergebnisse durchgeführter Einstellungen durch die Winkelmesser erfaßt und von der Anzeigevorrichtung angezeigt.

Eine Weiterbildung der Erfindung sieht vor, daß die Vorrichtung mit einem Navigationssystem zur Lageerfassung eines angefahrenen Zielpunktes ausgestattet ist, welche an der Anzeigeeinrichtung angezeigt wird. Es kann sich dabei beispielsweise um ein Kamerasystem oder Tastersystem zur Erfassung der Lage von Eichpunkten, Markierungen oder der am Arm befestigten Einrichtung selbst handeln. Es ist jedoch auch möglich, daß die Vorrichtung mit einem stereotaktischen Lokalisationssystem verknüpft ist, welches die Lage der Einrichtung und des Zielpunkts erfaßt. Als Lokalisationssystem sind alle Systeme möglich, zweckmäßigerweise wird eines ausgewählt, das ohne Schaden für den Patienten für längere Zeit betrieben werden kann, wie beispielsweise ein Magnetresonanztomograph. Die Lage der Einrichtung zum Zielpunkt kann auf einem Bildschirm angezeigt werden, wodurch der den Eingriff durchführende Arzt eine genaue Kontrolle hat.

Bei den vorgenannten Ausgestaltungen wurde auf Antriebe verzichtet und die Einstellungen wurden von Hand vorgenommen. Dies ist dadurch möglich, daß nach der Erfindung beim Einsatz für Stereotaxie die Einstellungen auch nacheinander vorgenommen werden können. Selbstverständlich schließt dies jedoch nicht aus, daß die Stellachsen mit Antrieben ausgestattet sind und ein Rechner mittels eines Programms zur selbsttätigen Einstellung der erforderlichen Winkelstellungen einrichtbar ist. Auch dabei bleibt der erfindungsgemäße Vorteil hoher Sicherheit und eines vertretbaren Rechen- und Steuerungsaufwands erhalten, da eine Position durch Einstellungen, die zeitlich nicht koordiniert werden müssen, angefahren wird. Weiterhin kann die Einstellung überprüft werden, bevor der eigentliche Eingriff in den Körper stattfindet. Letzterer vollzieht sich in einer Dimension und ist mit geringem Aufwand, präzise und mit hoher Sicherheit durchführbar. Die Einbringung der Einrichtung, beispielsweise einer Sonde kann von Hand erfolgen, gegebenenfalls über eine genau einstellbare Übersetzungsmechanik oder es kann vorgesehen sein, daß die Einrichtung mittels eines weiteren Antriebs auf dem Einbringweg zum Zielpunkt geführt wird.

Als Ergänzung zu solchen Antrieben wird dann vorgeschlagen, daß das Programm derart ausgebildet ist, daß es mittels des Rechners die Antriebe und gegebenenfalls einen weiteren, der Einrichtung zugeordneten Antrieb zur Durchführung eines diagnostischen oder therapeutischen Eingriffs veranlaßt. In diesem Fall erfolgt der Eingriff automatisch und die Aufgabe des Arztes besteht in der vorherigen Festlegung der Daten und der Überwachung des Eingriffs. Der Rechner wird dann zweckmäßigerweise mit einer Eingabeeinrichtung zur Steuerung des Eingriffs ausgestattet.

Die Arretiervorrichtungen können derart ausgebildet sein, daß sie nach Erreichung der Feineinstellungen von Hand betätigt werden müssen, oder es ist möglich, daß mit Erreichung der jeweiligen errechneten Sollposition die Arretiervorrichtungen die Stellachsen automatisch arretieren. Letzteres läßt sich in ein Programm für einen Rechner integrieren, welcher über die Winkelmesser die entsprechenden Ist-Daten erhält und diese mit den Soll-Daten vergleicht.

Zur weiteren Erhöhung der Sicherheit ist vorgesehen, daß die Vorrichtung für den Stereotaxieeinsatz mit einer Vorrichtung zur Abgabe eines Warnsignals ausgestattet ist, wenn eine der Einstellungen der Mechanik nicht mehr in ihrer Sollposition ist.

Soll die Vorrichtung zur Stereotaxie im Zusammenhang mit der Erfassung durch einen Magnetresonanztomographen eingesetzt werden, so wird vorgeschlagen, daß zumindest die vorderen Bereiche des Arms aus elektrisch nicht leitenden und unmagnetisierbaren Materialien hergestellt sind. Dabei kommen beispielsweise Faserverbundwerkstoffe oder Kunststoffe mit Glasfaserverstärkung in Betracht.

Die Bemessung der Länge des Arms kann für die Erreichung von Zielpunkten am gesamten menschlichen Körper bemessen sein. Es ist jedoch auch möglich, daß die Länge des Arms für die Erreichung von Punkten an einem Teilbereich des menschlichen Körpers, beispielsweise des Kopfes, bemessen ist. Auf diese Weise läßt sich der Arm wesentlich kürzer ausgestalten. Die Vorrichtung ist dann zwar nur noch für einen eingeschränkten Bereich einsetzbar, die Präzision ist jedoch durch die kürzere Ausgestaltung des Arms bedeutend höher, was gerade für den Einsatz im Kopfbereich wichtig sein kann. Der erfindungsgemäße Vorteil gegenüber dem eingangs genannten Stand der Technik bleibt dabei zu einem Teil trotzdem noch erhalten, da die erfindungsgemäße Ausgestaltung auch im Kopfbereich jede Einbringrichtung erlaubt, jeden beliebigen Markierungs- oder sonstigen Meßpunkt erreichen kann und nicht nur solche im oberen Schädelbereich.

Zur Erreichung eines hohen Sicherheitsstandards wird vorgeschlagen, daß der Vorrichtung ein Meßphantom zugeordnet ist, das einen Überprüfungszielpunkt aufweist, der in die Position des Zielpunktes gebracht werden kann und daß die Positionsbestimmungsvorrichtung die Erreichung des Überprüfungszielpunktes durch die maßgebliche Einrichtung erfaßt. Beispielsweise kann der Taster an der Stelle der maßgeblichen Einrichtung plaziert sein und dadurch als Positionsbestimmungsvorrichtung dienen. Bei Sonden ist der maßgebliche Sondenteil der Teil der Sonde, der den Zielpunkt erreichen und in entsprechender Weise bearbeiten soll. Solche Meßphantome können auf jede Weise ausgebildet sein, ein Beispiel dafür zeigt auch der eingangs genannte Stand der Technik. Mit einem solchen Meßphantom läßt sich auch eine Vorrichtung justieren, die dann der Patientenpositionierung, beziehungsweise der Verifikation derselben dient.

Das Ständerteil der Vorrichtung kann auch an der Decke über dem Patienten befestigbar sein. Um die Lage wählen zu können, kann eine entsprechende Vorrichtung, beispielsweise ein Kreuzschlitten, dienen.

Die Erfindung wird nachfolgend anhand eines in der Zeichnung dargestellten Ausführungsbeispiels erläutert. Es zeigen
- **Fig. 1**: ein Ausführungsbeispiel der Erfindung,
- **Fig. 2**: die Mechanik des Ausführungsbeispiels gemäß Fig. 1,
- **Fig. 3**: eine Vergrößerung des Eingriffsbereichs.

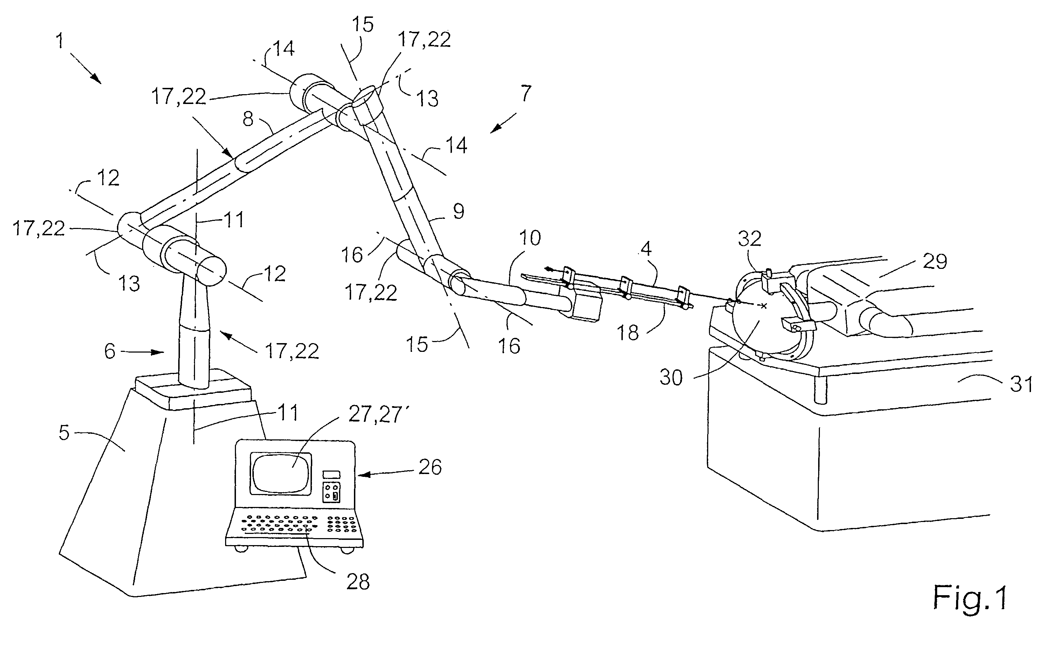

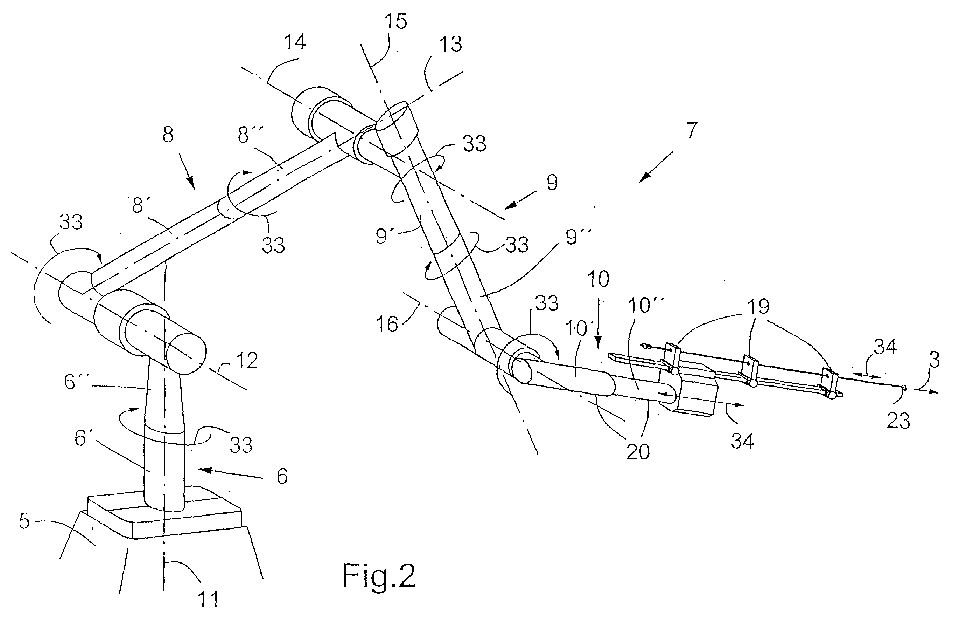

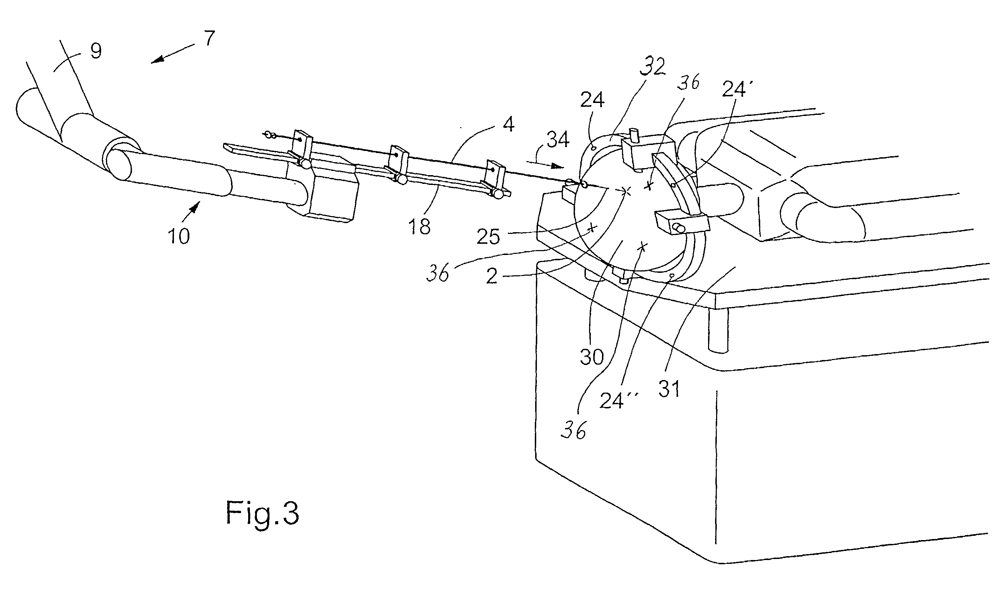

Die Darstellung der **Figuren 1, 2** und **3** zeigen dasselbe Ausführungsbeispiel der Erfindung, wobei gleiche Bezugszeichen identische Teile zeigen, der Übersichtlichkeit halber sind jedoch nicht alle Bezugszeichen in alle Darstellungen eingetragen. Dabei zeigt die **Fig. 1** eine Gesamtdarstellung des Ausführungsbeispiels des erfindungsgemäßen Gegenstandes mit der Mechanik 1, welche den Arm 7 aufweist, wobei ein Eingriff mittels einer Sonde 4 am Kopf 30 einer Person erfolgt. Der Arm 7 ist in **Fig. 2** vergrößert dargestellt, wobei zur Erläuterung weitere Einzelheiten eingetragen wurden. Der Eingriff selbst ist wiederum in **Fig. 3** vergrößert gezeichnet.

Die Mechanik 1 ist so ausgebildet, daß sie eine Sonde 4 derart an einen menschlichen Körper 29, beispielsweise am Kopf 30 positioniert, daß die Sonde 4 mittels einer Linearbewegung 34 zu einem Zielpunkt 2 gebracht werden kann. Zu diesem Zweck ist ein Ständer 5, 6 vorgesehen, der den aus mindestens drei Gliedern 8, 9 und 10 bestehenden Arm 7 trägt. Dabei ist vorgesehen, daß der Arm 7 mindestens fünf Stellachsen 11, 12, 13, 14, 15, 16 aufweist und diesen Stellachsen 11, 12, 13, 14, 15, 16 Feineinstell- und Arretiervorrichtungen 17 zugeordnet sind. Der Arm 7 ist mittels eines drehbaren Ständerteils 6 an einem Ständerteil 5 zur Fixierung auf dem Boden angelenkt. Der Ständer 5, 6 ist derart bezüglich Größe und Gewicht bemessen, daß er im Bereich des Patienten 29 beliebig plazierbar ist und bei ärztlichen Maßnahmen nicht im Weg ist oder den Raum für weitere Geräte verstellt. Er muß jedoch trotzdem absolute Standsicherheit aufweisen, diesbezüglich wird auf die obigen Ausführungen verwiesen.

Das Ständerteil 6 besteht aus zwei gegenseitig drehbaren Teilen 6' und 6", welche eine Drehbewegung 33 zulassen. Dadurch kann der Arm 7 innerhalb eines Raums geschwenkt werden. An das Ständerteil 6 zur Anlenkung des Arms 7 ist ein Glied 8 des Arms 7 angelenkt, wobei die Anlenkung eine weitere als Drehachse ausgebildete Stellachse 12 aufweist, die ebenfalls eine Drehbewegung 33 senkrecht zur ersten Drehbewegung 33 zuläßt. Auch diese Stellachse 12 wie alle weiteren Stellachsen 13, 14, 15, 16 können die jeweils eingezeichneten Drehbewegungen 33 vollziehen und sind mit Feineinstell- und Arretiervorrichtungen 17 ausgestattet. Auch das am Ständer 5, 6 befestigte Glied 8 weist zwei drehbare Teile 8' und 8" auf und das sich daran anschließende Zwischenglied 9 zwei drehbare Teile 9' und 9". Die Anlenkung des Zwischenglieds 9 an das Glied 8 ist eine ebenfalls als Drehachse ausgebildete Stellachse 14, genauso die Anlenkung des vorderen Gliedes 10 an das Zwischenglied 9 mittels der Stellachse 16. Das vordere Glied 10 kann dagegen aus zwei teleskopartig verschiebbaren Teilen 10' und 10" bestehen, welche eine Führung 20 für eine Linearbewegung 34 zur Einbringung einer Sonde 4 bilden. Alternativ kann auch ein Sondenhalter 18 eine Führung 19 mit demselben Zweck aufweisen.

Statt einer Sonde 4 läßt sich auch eine andere Einrichtung 4 am Arm 7 anbringen, beispielsweise ein Taster zur Erfassung von Markierungen 36 am Patienten 29, beispielsweise am Kopf 30, wie dies in Fig. 3 dargestellt ist. Werden die Markierungen 36 angefahren und ihre Lage erfaßt, so kann die exakte Lage des Patienten 29 oder eines Körperteils, wie der Kopf 30, erfaßt werden und es kann eine Lagekorrektur, vorzugsweise eine Rechenkorrektur vorgenommen werden, um danach einen Zielpunkt 2 exakt anfahren zu können.

Um die Sonde 4 in die Eingriffsposition zu verbringen, in der sie in Eingriffsrichtung 3 nach Durchbrechung der Körperoberfläche den Einbringweg 25 zu einem Zielpunkt 2 zurücklegt, muß eine entsprechende Positionierung der Sonde 4 vor dem Körper 29 erfolgen. Diese Position wird durch den Zielpunkt 2 und die Richtung 3 definiert. Dazu wird die Mechanik 1 mit dem Ständer 5 derart positioniert, daß sie die Vornahme weiterer ärztlicher Maßnahmen möglichst wenig behindert, jedoch ein aus medizinischer Sicht optimaler Einbringweg 25 möglich ist. Nach der Positionierung des Ständers 5 wird in den Sondenhalter 18 ein Taster 23 eingelegt und eine Eichung des Arms 7 vorgenommen. Zu diesem Zweck trägt eine Fixiervorrichtung 32 für den menschlichen Körper 29, hier beispielsweise der Kopf 30, drei Eichpunkte 24, 24', 24", welche mittels des Tasters 23 angefahren werden. Der Taster 23 gibt in den entsprechenden Positionen ein Signal und die den Stellachsen 11 bis 16 zugeordneten Winkelmesser 22 geben die zugehörigen Winkelpositionen an einen Rechner 26, welcher dadurch die genaue Position der Mechanik 1 mit dem des Ständers 5, 6 und dem Arm 7 ermitteln kann.

Verbleibt eine solche Fixiervorrichtung 32 zwischen einer Bilderfassung und dem Eingriff am Patienten 29, so können die Eichpunkte 24, 24', 24" als Markierungen 36 zur Patientenpositionierung dienen. Oder es ist mittels zusätzlicher Markierungen 36 eine Verifikation der Lage der Fixiervorrichtung 32 möglich, indem deren Lage mittels der Eichpunkte 24, 24', 24" erfaßt und mit der Lage der Markierungen 36 verglichen wird.

Eine Bedienperson gibt bei einem stereotaktischen Eingriff mittels einer Eingabeeinrichtung 28 die zuvor festgelegten Daten vom Zielpunkt 2 sowie der Einbringrichtung 3, welche den medizinisch günstigsten Einbringweg 25 beinhaltet, ein. Daraufhin berechnet der Rechner 26 durch ein entsprechendes Programm die notwendigen Winkelstellungen, die an den Stellachsen 11 bis 16 eingestellt werden müssen. Dabei wird mit der Stellachse 11 der Arm 7 in die richtige Position im Raum gebracht und es erfolgt mittels der Stellachsen 12, 14 und 16 eine Einstellung der Entfernung. Der entsprechende Winkel im Raum wird dann mittels der Stellachsen 13, 15 und 16 angefahren.

Dies ist ein komplizierter Einstellvorgang, welcher ohne Zeitverlust nur dadurch vornehmbar ist, daß der Rechner 26 die Einstelldaten ermittelt und an einer Anzeigeeinrichtung 27, beispielsweise einem Bildschirm 27' anzeigt, so daß eine Bedienperson in der Lage ist, diese Einstellungen an einer Stellachse 11 bis 16 nach der anderen vorzunehmen.

Zweckmäßigerweise ist der Arm 7 an allen Stellachsen 11 bis 16 mit Winkelmessern 22 ausgestattet, die mit dem Rechner 26 verbunden sind, wodurch genaue Anzeigen der Istpositionen und vorzugsweise auch der Sollpositionen auf dem Bildschirm 27 möglich sind. Vorteilhafterweise werden die Feineinstell- und Arretiervorrichtungen 17 vom Rechner 26 mit Erreichen der Sollposition automatisch arretiert. Sind alle Einstellungen erfolgt, so wird, wenn ein Taster 23 als Positionsbestimmungsvorrichtung verwendet wurde, dieser durch eine Sonde 4 ersetzt und gegebenenfalls vor oder nach dieser Ersetzung nochmals die Position genauestens überprüft.

Danach kann der in Fig. 3 dargestellte Eingriff vorgenommen werden, indem beispielsweise der Arzt die Sonde 4 durch die Körperoberfläche hindurch zum Zielpunkt 2 führt, gegebenenfalls unter Vornahme einer Bohrung durch die Schädeldecke, und dort die Behandlung oder Diagnosehandlung vornimmt. Die jeweilige Position der Sonde 4 kann dabei erfaßt und auf dem Bildschirm 27' dargestellt werden, um eine genaue Überwachung zu ermöglichen.

Beim Einsatz als Positioniervorrichtung müssen Punkte wie Markierungen 36 oder Eichpunkte 24, 24', 24" angefahren werden, um deren Koordinaten zu bestimmen, womit auch die Lage des Patienten 29 oder eines Körperteils 30 bestimmt ist und dann ein Zielpunkt 2 exakt angefahren werden kann.

Die Darstellungen sind lediglich ein Ausführungsbeispiel. Der Ständer 5 kann auch eine Befestigungsplatte sein, mit der ein relativ klein ausgebildeter Arm 7 direkt an einer Fixiervorrichtung 32, beispielsweise einem Kopfhalterungsring, befestigbar ist und der Erreichung eines Zielpunktes 2 im Kopf 30 dient. Weitere Ausgestaltungen des Ständerteils 5 mit zeichnerisch nicht weiter dargestellten Einrichtungen zur Unterbindung einer ungewollten Standortveränderung sind möglich. Bezüglich der weiteren nicht dargestellten Ausgestaltungsmöglichkeiten wird auf die allgemeine Beschreibung der Erfindung verwiesen.

Die Vorrichtung läßt sich auch ausschließlich als Positionier- und Verifikationsvorrichtung einsetzen, um die exakte Lage eines Zielpunktes 2 zum Zweck eines anderweitig durchgeführten Eingriffs, wie einer Bestrahlung, zu überprüfen und gegebenenfalls die Lage zu korrigieren, beziehungsweise den Eingriff entsprechend der ermittelten Lage vorzunehmen.

### Bezugszeichenliste

- 1: Mechanik
- 2: Zielpunkt
- 3: Richtung
- 4: Einrichtung, z. B. Sonde (für medizinischen Eingriff, Untersuchung oder Positionsmessung)
- 5,6: Ständer
- 5: Ständerteil zur Fixierung auf dem Boden
- 6, 6', 6": Ständerteil zur Anlenkung des Arms
- 6', 6": drehbare Teile des Ständerteils zur Anlenkung des Arms
- 7: Arm
- 8, 9, 10: Glieder des Arms
- 8: am Ständer angeordnetes Glied
- 8', 8": drehbare Teile des am Ständer angeordneten Glieds
- 9: Zwischenglied
- 9', 9": gegenseitig drehbare Teile des Zwischenglieds
- 10: vorderes Glied
- 10', 10": gegenseitig teleskopartig verschiebbare Teile des vorderen Gliedes
- 11 bis 16: Stellachsen
- 11: Stellachse der drehbaren Teile des Ständerteils zur Anlenkung des Arms
- 12: Stellachse zwischen dem Ständerteil zur Anlenkung des Arms und dem Arm
- 13: Stellachse der drehbaren Teile des am Ständer angeordneten Glieds des Arms
- 14: Stellachse zwischen dem am Ständer angeordneten Glied und dem Zwischenglied
- 15: Stellachse der drehbaren Teile des Zwischenglieds
- 16: Stellachse zwischen Zwischenglied und vorderem Glied
- 17: Feineinstell- und Arretiervorrichtungen
- 18: Halter, z. B. Sondenhalter
- 19: Führung am Sondenhalter
- 20: Führung am vorderen Glied des Arms
- 21: weitere Stellachse
- 22: Winkelmesser
- 23: Taster
- 24, 24', 24": drei Eichpunkte
- 25: Einbringweg
- 26: Rechner
- 27, 27': Anzeigeeinrichtung
- 27': Bildschirm
- 28: Eingabeeinrichtung
- 29: Patient (gesamter menschlicher Körper)
- 30: Kopf
- 31: Patiententisch
- 32: Fixiervorrichtung (z. B. Kopfhalterungsring)
- 33: Pfeile: Drehbewegungen
- 34: Pfeile: Linearbewegung
- 35: Pfeile: Stellbewegungen dreier Achsen zur Erzielung einer gradlinigen Sondenbewegung
- 36: Markierungen am Patienten, z. B. am Kopf

## Patentansprüche

1. Medizinische Vorrichtung mit einer Mechanik (1) zur Positionierung einer Einrichtung (4) mittels eines Armes (7), welcher aus mindestens drei Gliedern (8, 9, 10) besteht, wobei die Mechanik (1) mindestens fünf aufeinanderfolgende Stellachsen (11,12, 13, 14, 15, 16) aufweist, welchen separate Feineinstell- und Arretiervorrichtungen (17) zugeordnet sind, wobei die Mechanik ( 1 ) ein Ständerteil (5) zur Fixierung an einem beliebigen Standort im Bereicht des Patienten (29) aufweist,
**dadurch gekennzeichnet,**
**daß** jede Stellachse (11, 12, 13, 14, 15, 16) eine Drehbewegung (33) zuläßt, die senkrecht zur Drehbewegung (33) der jeweils unmittelbar angrenzenden Stellachse (11, 12, 13, 14, 15 oder 16) ausgerichtet ist, derart, daß die Einrichtung (4) in nahezu jeder beliebigen Richtung (3) jeden beliebigen Zielpunkt (2) im oder am Patienten (29) erreichen kann.

2. Vorrichtung nach Anspruch 1,
**dadurch gekennzeichnet,**
**daß** das Ständerteil (5) eine Einrichtung zur Unterbindung einer ungewollten Standortveränderung aufweist.

3. Vorrichtung nach Anspruch 2,
**dadurch gekennzeichnet,**
**daß** das Ständerteil (5) auf dem Boden im Bereich des Patienten (29) fixierbar ist.

4. Vorrichtung nach Anspruch 1, 2 oder 3
**dadurch gekennzeichnet,**
**daß** das Ständerteil (5) so klein ausgestaltet ist, daß es noch genügend Standsicherheit gewährleistet, ansonsten jedoch keinen Raum beansprucht, der für ärztliche Maßnahmen oder weitere Geräte zur Verfügung stehen muß.

5. Vorrichtung nach Anspruch 3 oder 4,
**dadurch gekennzeichnet,**
**daß** das Ständerteil (5) Rollen und eine Einrichtung zur Unterbindung der Rollenbewegung aufweist.

6. Vorrichtung nach Anspruch 2,
**dadurch gekennzeichnet,**
**daß** das Ständerteil (5) mit einem Teil verbindbar ist, das einen festen Bezug zum Patienten (29) aufweist.

7. Vorrichtung nach Anspruch 6,
**dadurch gekennzeichnet,**
**daß** das Ständerteil (5) mit einer Fixiervorrichtung (32) verbindbar ist.

8. Vorrichtung nach Anspruch 6,
**dadurch gekennzeichnet,**
**daß** das Ständerteil (5) mit dem Patiententisch (31) verbindbar ist.

9. Vorrichtung nach einem der Ansprüche 1 bis 8,
**dadurch gekennzeichnet,**
**daß** eine weitere sechste Stellachse (11, 12, 13, 14, 15 oder 16) vorgesehen ist, der ebenfalls eine Feineinstell- und Arretiervorrichtung (17) zugeordnet ist.

10. Vorrichtung nach einem der Ansprüche 1 bis 9,
**dadurch gekennzeichnet,**
**daß** das vorderste Glied (10) mit einem Halter (18) zur Aufnahme verschiedenster Einrichtungen (4) ausgestattet ist.

11. Vorrichtung nach Anspruch 10,
**dadurch gekennzeichnet,**
**daß** der Halter (18) für die geradlinige Bewegung einer Einrichtung (4) eine in Richtung (3) auf einen Zielpunkt (2) richtbare Führung (19) aufweist.

12. Vorrichtung nach Anspruch 11,
**dadurch gekennzeichnet,**
**daß** die Führung (19) des Halters (18) in einem Winkel zur Ausrichtung des vordersten Glieds (10) verläuft.

13. Vorrichtung nach einem der Ansprüche 1 bis 10,
**dadurch gekennzeichnet,**
**daß** das vordere Glied (10) des Arms (7) für die geradlinige Bewegung einer Einrichtung (4) eine in Richtung (3) auf einen Zielpunkt (2) richtbare Führung (20) aufweist.

14. Vorrichtung nach einem der Ansprüche 1 bis 10,
**dadurch gekennzeichnet,**
**daß** für die geradlinige Bewegung einer Einrichtung (4) eine weitere Stellachse (21) vorgesehen ist, die zu zwei Stellachsen (14, 16), deren Glieder (9, 10) in derselben oder parallelen Ebenen bewegbar sind, eine derartige Kopplung aufweist, daß die Stellachsen (21, 14, 16) gegenläufige Winkelstellungen in sich gegenseitig aufhebender Weise vollziehen und dabei gleichzeitig eine Korrektur zum Verbleib der Lage der Einrichtung (4) in Richtung (3) auf einen Zielpunkt (2) durchführen können.

15. Vorrichtung nach einem der Ansprüche 1 bis 14,
**dadurch gekennzeichnet,**
**daß** die Stellachsen (11, 12, 13, 14, 15, 16 und ggf. 21) mit Winkelmessern (22) ausgestattet sind.

16. Vorrichtung nach einem der Ansprüche 1 bis 15,
**dadurch gekennzeichnet,**
**daß** sie mit einem Rechner (26) verbindbar ist und ein Programm für einen Rechner (26) aufweist, das über die Winkelstellungen der Stellachsen (11, 12, 13, 14, 15, 16 und ggf. 21) einen frei wählbaren Zielpunkt (2) und einen frei wählbaren Standort des Ständers (5, 6) derart zueinander in Bezug setzt, daß einer davon aufgrund bekannter Koordinaten des anderen errechenbar ist.

17. Vorrichtung nach Anspruch 16,
**dadurch gekennzeichnet,**
**daß** eine Einrichtung zur Bestimmung der Koordinaten beliebiger Standorte des Ständers (5, 6) vorgesehen ist.

18. Vorrichtung nach Anspruch 16 in Kombination mit 17,
**dadurch gekennzeichnet,**
**daß** sie ein Programm für einen Rechner (26) aufweist, das für Standorte des Ständers (5, 6) die Winkelstellungen der Stellachsen (11, 12, 13, 14, 15, 16 und ggf. 21) zur Erreichung eines Zielpunktes (2) errechnet.

19. Vorrichtung nach Anspruch 16 in kombination mit 17,
**dadurch gekennzeichnet,**
**daß** sie ein Programm für einen Rechner (26) aufweist, das aufgrund des Standorts des Ständers (5,6) und der Winkelstellungen der Stellachsen (11, 12, 13, 14, 15, 16 und ggf. 21) die Position eines durch eine Einrichtung (4) angefahrenen Zielpunktes (2) errechnet.

20. Vorrichtung nach Anspruch 17, 18 oder 19,
**dadurch gekennzeichnet,**
**daß** der Arm (7) mit einer Positionsbestimmungsvorrichtung ausgestattet ist.

21. Vorrichtung nach einem der Ansprüche 17 bis 20,
**dadurch gekennzeichnet,**
**daß** sie ein Programm für einen Rechner (26) aufweist, das mittels dreier als Zielpunkte (2) definierter Eichpunkte (24, 24', 24") die Lage beliebiger gewählter Standorte des Ständers (5, 6) bestimmt.

22. Vorrichtung nach Anspruch 21,
**dadurch gekennzeichnet,**
**daß** die Eichpunkte (24, 24', 24") einen definierten Bezug zum Patienten (29) aufweisen und daher die Relativlage des Ständers (5, 6) zum Patienten (29) bestimmbar ist.

23. Vorrichtung nach Anspruch 21 oder 22,
**dadurch gekennzeichnet,**
**daß** das Programm nach der Bestimmung des Standortes des Ständers (5, 6) mittels der Eichpunkte (24, 24', 24") die Winkelstellungen der Stellachsen (11, 12, 13, 14, 15, 16 und ggf. 21) zur Erreichung eines vorgegebenen Zielpunktes (2) ermittelt.

24. Vorrichtung nach Anspruch 21 oder 22,
**dadurch gekennzeichnet,**
**daß** das Programm nach der Bestimmung der Standorte des Ständers (5, 6) mittels der Eichpunkte (24, 24', 24") aus den Winkelstellungen der Stellachsen (11, 12, 13, 14, 15, 16 und ggf. 21) die Lage mindestens eines angefahrenen Zielpunktes (2) überprüft.

25. Vorrichtung nach Anspruch 24,
**dadurch gekennzeichnet,**
**daß** die Lage mehrerer als Zielpunkte (2) dienender Markierungen (36) am Patienten (29) oder Markierungen (36, 24, 24', 24") mit festem Bezug zum Patienten (29) zur Bestimmung und/oder Überprüfung von dessen Lage dienen.

26. Vorrichtung nach einem der Ansprüche 18 bis 23,
**dadurch gekennzeichnet,**
**daß** das Programm auch den Einbringweg (25) bis zur Erreichung eines im Körper eines Patienten (29) liegenden Zielpunktes (2) ermittelt.

27. Vorrichtung nach Anspruch 16 bis 26,
**dadurch gekennzeichnet,**
**daß** sie mit einem Rechner (26) ausgestattet ist, welcher zur Bestimmung der Lage mindestens eines Zielpunktes (2) zum Standort des Ständers (5,6) oder umgekehrt mit einem Programm geladen ist.

28. Vorrichtung nach einem der Ansprüche 17 bis 27,
**dadurch gekennzeichnet,**
**daß** die Winkelmesser (22) und die Positionsbestimmungsvorrichtung mit einem Rechner (26) verbunden sind, wobei dieser die Daten des Standorts des Ständers (5, 6), der Einstellungen der Mechanik (1) und die Daten eines Zielpunktes (2) ermittelt.

29. Vorrichtung nach Anspruch 28,
**dadurch gekennzeichnet,**
**daß** die Ermittelung der Daten der Erreichung eines vorgegebenen Zielpunktes (2) dient.

30. Vorrichtung nach Anspruch 28,
**dadurch gekennzeichnet,**
**daß** die Ermittelung der Daten der Bestimmung der Lage eines angefahrenen Zielpunktes (2) dient.

31. Vorrichtung nach einem der Ansprüche 17 bis 30,
**dadurch gekennzeichnet,**
**daß** das Programm derart ausgebildet ist, daß ein Rechner (26) die ermittelten Daten an einer Anzeigeeinrichtung (27, 27') anzeigt.

32. Vorrichtung nach Anspruch 29 oder 31,
**dadurch gekennzeichnet,**
**daß** auch die Einstellergebnisse durchgeführter Einstellungen durch die Winkelmesser (22) erfaßt und von der Anzeigeeinrichtung (27, 27') angezeigt werden.

33. Vorrichtung nach einem der Ansprüche 1 bis 32,
**dadurch gekennzeichnet,**
**daß** sie mit einem Navigationssystem zur Lageerfassung eines angefahrenen Zielpunktes (2) der Sonde (4) ausgestattet ist, welche an einer bzw. der Anzeigeeinrichtung (27, 27') angezeigt wird.

34. Vorrichtung nach Anspruch 33,
**dadurch gekennzeichnet,**
**daß** sie mit einem Kamerasystem oder Tastersystem zur Erfassung eines mit der Einrichtung (4) angefahrenen Zielpunktes (2) ausgestattet ist.

35. Vorrichtung nach einem der Ansprüche 1 bis 34,
**dadurch gekennzeichnet,**
**daß** es mit einem stereotaktischcn Lokalisationssystem verknüpft ist, welches die Lage der Einrichtung (4) zum Zielpunkt (2) erfaßt.

36. Vorrichtung nach Anspruch 35,
**dadurch gekennzeichnet,**
**daß** die Lage der Einrichtung (4) zum Zielpunkt (2) auf einem Bildschirm (27') angezeigt wird.

37. Vorrichtung nach einem der Ansprüche 16 bis 36,
**dadurch gekennzeichnet,**
**daß** die Stellachsen (11, 12, 13, 14, 15, 16) mit Antrieben ausgestattet sind und ein Rechner (26) mittels eines Programms zur selbsttätigen Einstellung der erforderlichen Winkelstellungen einrichtbar ist.

38. Vorrichtung nach Anspruch 37,
**dadurch gekennzeichnet,**
**daß** mittels eines weiteren Antriebs die Einrichtung (4) auf dem Einbringweg (25) zum Zielpunkt (2) geführt werden kann.

39. Vorrichtung nach Anspruch 38,
**dadurch gekennzeichnet,**
**daß** das Programm derart ausgebildet ist, daß es mittels des Rechners (26) die Antriebe und gegebenenfalls mindestens einen weiteren, der Einrichtung (4) zugeordneten Antrieb, zur Durchführung eines diagnostischen oder therapeutischen Eingriffs veranlaßt.

40. Vorrichtung nach Anspruch 39,
**dadurch gekennzeichnet,**
**daß** der Rechner (26) mit einer Eingabeeinrichtung (28) zur Steuerung des Eingriffs ausgestattet ist.

41. Vorrichtung nach einem der Ansprüche 16 bis 40,
**dadurch gekennzeichnet,**
**daß** mit Erreichung der jeweiligen errechneten Sollposition die Feineinstell- und Arretiervorrichtungen (17) die Stellachsen (11 bis 16) automatisch arretieren.

42. Vorrichtung nach einem der Ansprüche 16 bis 41,
**dadurch gekennzeichnet,**
**daß** sie mit einer Vorrichtung zur Abgabe eines Warnsignals ausgestattet ist, wenn eine der Einstellungen der Mechanik (1) nicht mehr in ihrer Sollposition ist.

43. Vorrichtung nach einem der Ansprüche 1 bis 42,
**dadurch gekennzeichnet,**
**daß** zumindest die vorderen Bereiche des Arms (7) aus elektrisch nichtleitenden und unmagnetisierbaren Materialien hergestellt sind.

44. Vorrichtung nach einem der Ansprüche 1 bis 43,
**dadurch gekennzeichnet,**
**daß** die Länge des Arms (7) für die Erreichung von Zielpunkten (2) am gesamten menschlichen Körper (29) bemessen ist.

45. Vorrichtung nach einem der Ansprüche 1 bis 43,
**dadurch gekennzeichnet,**
**daß** die Länge des Arms (7) für die Erreichung von Zielpunkten (2) an einem Teilbereich des menschlichen Körpers (29) bemessen ist.

46. Vorrichtung nach Anspruch 45,
**dadurch gekennzeichnet,**
**daß** die Länge des Arms (7) für die Erreichung von Zielpunkten am Kopf (29) bemessen ist.

47. Vorrichtung nach einem der Ansprüche 1 bis 46,
**dadurch gekennzeichnet,**
**daß** ihr ein Meßphantom zugeordnet ist, das einen Überprüfungszielpunkt aufweist, der in die Position eines Zielpunktes (2) gebracht werden kann und daß eine Positionsbestimmungsvorrichtung die Erreichung des Überprüfungszielpunktes erfaßt.

48. Vorrichtung nach Anspruch 47,
**dadurch gekennzeichnet,**
**daß** ein Taster (23) als Einrichtung (4) plazierbar ist und als Positionsbestimmungsvorrichtung dient.

49. Vorrichtung nach einem der Ansprüche 1 bis 48,
**dadurch gekennzeichnet,**
**daß** das Ständerteil (5) an der Decke über dem Patienten (29) befestigbar ist.

## Claims

1. A medical device comprising a mechanical system (1) for positioning a contrivance (4) by means of an arm (7) composed of at least three members (8, 9, 10), which mechanical system (1) has at least five successive adjustment axes (11, 12, 13, 14, 15, 16) to which separate fine adjustment and arresting means (17) are assigned, said mechanical system (1) having a pedestal component (5) capable of being fixed in any desired position in the vicinity of a patient (29), **characterized in that** each adjustment axis (11, 12, 13, 14, 15, 16) allows for rotary motion (33) oriented at right angles to the rotary motion (33) of the adjoining adjustment axes (11, 12, 13, 14, 15 or 16) such that said contrivance (4) can be moved in almost any desired direction (3) toward any desired target (2) in or on said patient (29).

2. A device as defined in claim 1, **characterized in that** said pedestal component (5) has means for the prevention of an unintentioned change of position.

3. A device as defined in claim 2, **characterized in that** said pedestal component (5) is capable of being fixed in position on the floor in the vicinity of said patient (29).

4. A device as defined in claim 1, 2 or 3, **characterized in that** said pedestal component (5) is small enough to ensure adequate stability of position whilst not occupying space otherwise required for medical purposes or for other apparatus.

5. A device as defined in claim 3 or claim 4, **characterized in that** said pedestal component (5) has rollers and means for preventing movement of said rollers.

6. A device as defined in claim 2, **characterized in that** said pedestal component (5) can be connected to an item bearing a fixed relationship to said patient (29).

7. A device as defined in claim 6, **characterized in that** said pedestal component (5) can be connected to an arresting device (32).

8. A device as defined in claim 6, **characterized in that** said pedestal component (5) can be connected to the patient's table (31).

9. A device as defined in any one of claims 1 to 8, **characterized in that** another, sixth, adjustment axis (11, 12, 13, 14, 15 or 16) is provided to which likewise fine adjustment and arresting means (17) are assigned.

10. A device as defined in any one of claims 1 to 9, **characterized in that** the foremost member (10) is equipped with a holder (18) for accommodation of a large variety of contrivances (4).

11. A device as defined in claim 10, **characterized in that** said holder (18) exhibits, for the purpose of effecting linear movement of said contrivance (4), a guide (19) that is capable of being aligned in direction (3) toward said target (2).

12. A device as defined in claim 11, **characterized in that** said guide (19) of said holder (18) is aligned at an angle to the direction of alignment of said foremost member (10).

13. A device as defined in any one of claims 1 to 10, **characterized in that** said foremost member (10) of said arm (7) exhibits, for the purpose of effecting linear movement of said contrivance (4), a guide (20) that can be aligned in direction (3) toward said target (2).

14. A device as defined in any one of claims 1 to 10, **characterized in that**, for effecting linear motion of a contrivance (4), there is provided another adjustment axis (21) which is coupled to two adjustment axes (14, 16), whose members (9, 10) can be moved in the same or parallel planes, such that said adjustment axes (21, 14, 16) take up contrary angular positions with mutual compensation whilst simultaneously being able to effect corrective measures for maintaining the position of said contrivance (4) aligned in direction (3) toward said target (2).

15. A device as defined in any one of claims 1 to 14, **characterized in that** said adjustment axes (11, 12, 13, 14, 15, 16 and possibly 21) are equipped with angular position measuring devices (22).

16. A device as defined in any one of claims 1 to 15, **characterized in that** it can be connected to a computer (26) and includes a program for a computer (26) which can, by implementing the angular positions of said adjustment axes (11, 12, 13, 14, 15, 16 and possibly 21), relate a freely selectable target (2) to a freely selectable location of said pedestal (5, 6) in such a manner that the one can be computed on the basis of the known coordinates of the other.

17. A device as defined in claim 16, **characterized in that** there is provided a system for determining the coordinates of arbitrary locations of said pedestal (5, 6).

18. A device as defined in claim 16 in conjunction with claim 17, **characterized in that** it includes a program for a computer (26) which, for a given location of said pedestal (5, 6), computes the angular positions of said adjustment axes (11, 12, 13, 14, 15, 16 and possibly 21) that are necessary for reaching said target (2).

19. A device as defined in claim 16 in conjunction with claim 17, **characterized in that** it includes a program for a computer (26) which can, on the basis of the location of said pedestal (5, 6) and the angular positions of said adjustment axes (11, 12, 13, 14, 15, 16 and possibly 21 ), compute the position of said target (2) approached by said contrivance (4).

20. A device as defined in claim 17, 18 or claim 19, **characterized in that** said arm (7) is equipped with a positioning device.

21. A device as defined in any one of claims 17 to 20, **characterized in that** it includes a program for a computer (26) which determines, on the basis of three calibration marks (24, 24', 24") defined as targets (2), the position of arbitrarily selected locations of said pedestal (5, 6).

22. A device as defined in claim 21, **characterized in that** said calibration marks (24, 24', 24") bear a defined relationship to said patient (29), so that it is possible to determine the position of said pedestal (5, 6) relative to said patient (29).

23. A device as defined in claim 21 or claim 22, **characterized in that** said program, following determination of the location of said pedestal (5, 6) by means of said calibration marks (24, 24', 24"), computes the angular positions of said adjustment axes (11, 12, 13, 14, 15, 16 and possibly 21) as required for reaching a given target (2).

24. A device as defined in claim 21 or claim 22, **characterized in that** said program, following determination of the location of said pedestal (5, 6) on the basis of said calibration marks (24, 24', 24"), checks the position of at least one desired target (2) on the basis of the angular positions of said adjustment axes (11, 12, 13, 14, 15, 16 and possibly 21).

25. A device as defined in claim 24, **characterized in that** the positions of a plurality of marks (36) on said patient (29) serving as targets (2) or marks (36, 24, 24', 24") bearing a fixed relationship to said patient (29) serve to determine and/or check the position of said patient.

26. A device as defined in any one of claims 18 to 23, **characterized in that** said program also determines the path (25) leading to a target (2) within the body of said patient (29).

27. A device as defined in any one of claims 16 to 26, **characterized in that** it is equipped with a computer (26) in which a program is installed which is capable of determining the position of at least one target (2) relative to the location of said pedestal (5, 6) or vice versa.

28. A device as defined in any one of claims 17 to 27, **characterized in that** said angular position measuring devices (22) and said positioning device are connected to a computer (26) which determines the data of location of said pedestal (5, 6), the settings of said mechanical system (1), and the data of said target (2).

29. A device as defined in claim 28, **characterized in that** the determination of said data serves the purpose of reaching a given target (2).

30. A device as defined in claim 28, **characterized in that** the determination of said data serves the purpose of determining the position of an approached target (2).

31. A device as defined in any one of claims 17 to 30, **characterized in that** said program is designed in such a manner that a computer (26) will display the computed data on a display device (27, 27').

32. A device as defined in claim 29 or claim 31, **characterized in that** the values achieved by adjustments effected by said angular position measuring devices (22) are likewise registered and will be displayed on said display device (27, 27').

33. A device as defined in any one of claims 1 to 32, **characterized in that** it is equipped with a navigation system for finding the position of said target (2) approached by said probe (4), which position is indicated on said display device (27, 27') or by some other display means.

34. A device as defined in claim 33, **characterized in that** it is equipped with a camera system or sensor system for scanning a target (2) approached by said contrivance (4).

35. A device as defined in any one of claims 1 to 34, **characterized in that** it is linked to a stereotactic localization system, which will register the position of said contrivance (4) relative to said target (2).

36. A device as defined in claim 35, **characterized in that** the position of said contrivance (4) relative to said target (2) is displayed on a monitor (27').

37. A device as defined in any one of claims 16 to 36, **characterized in that** said adjustment axes (11, 12, 13, 14, 15, 16) are equipped with drives, and a computer (26) can be adapted by means of a program so as to automatically set the necessary angular positions.

38. A device as defined in claim 37, **characterized in that** said contrivance (4) can be guided by means of a further drive to enable it to be moved along path (25) toward said target (2).

39. A device as defined in claim 38, **characterized in that** said program is designed in such a manner that said drives and optionally at least one further drive assigned to said contrivance (4) can be caused to execute a diagnostic or therapeutic operation with the aid of said computer (26).

40. A device as defined in claim 39, **characterized in that** said computer (26) is equipped with an input device (28) for the purpose of controlling said operation.

41. A device as defined in any one of claims 16 to 40, **characterized in that** when the computed correct position is reached by said fine adjustment and arresting means (17), said adjustment axes (11-16) are automatically arrested.

42. A device as defined in any one of claims 16 to 41, **characterized in that** it is equipped with a device for emitting a warning signal when any one of the settings of said mechanical system (1) is no longer correct.

43. A device as defined in any one of claims 1 to 42, **characterized in that** at least the front regions of said arm (7) are composed of electrically non-conductive and non-magnetizable materials.

44. A device as defined in any one of claims 1 to 43, **characterized in that** the length of said arm (7) is dimensioned so as to enable it to reach targets (2) over the entire human body (29).

45. A device as defined in any one of claims 1 to 43, **characterized in that** the length of said arm (7) is dimensioned so as to enable it to reach targets (2) in a delimited region of the human body (29).

46. A device as defined in claim 45, **characterized in that** the length of said arm (7) is dimensioned so as to enable it to reach targets on the head (29).

47. A device as defined in any one of claims 1 to 46, **characterized in that** it is associated with a test phantom, which exhibits a check target capable of being moved to the position of said target (2) and that a positioning device detects when said check target is reached.

48. A device as defined in claim 47, **characterized in that** a sensor (23) can be inserted to act as said contrivance (4) to serve as positioning device.

49. A device as defined in any one of claims I to 48, **characterized in that** said pedestal component (5) can be fixed to the ceiling above said patient (29).

## Revendications

1. Dispositif médical comportant une mécanique (1) pour le positionnement d'un dispositif (4) au moyen d'un arbre (7) qui se compose au moins de trois organes (8, 9, 10), la mécanique (1) présentant au moins cinq axes de réglage consécutifs (11, 12, 13, 14, 15, 16) qui sont associés à des appareils de réglage fin et de blocage (17), la mécanique (1) présentant un pied (5) pour la fixation à n'importe quel endroit à proximité du patient (29), **caractérisé en ce que** chaque axe de réglage (11, 12, 13, 14, 15 ou 16) permet un mouvement rotatif (33) qui est orienté perpendiculairement au mouvement de rotation (33) de l'axe de réglage respectif à proximité immédiate (11, 12, 13, 14, 15 ou 16), de sorte que le dispositif (4) puisse atteindre dans pratiquement toutes les directions (3) n'importe quelle cible (2) dans ou sur le patient (29).

2. Dispositif selon la revendication 1, **caractérisé en ce que** le pied (5) présente un dispositif pour empêcher un changement de lieu involontaire.

3. Dispositif selon la revendication 3, **caractérisé en ce que** le pied (5) peut être fixé sur le sol dans la zone du patient (29).

4. Dispositif selon la revendication 1, 2 ou 3, **caractérisé en ce que** le pied (5) est réalisé dans une dimension de petite taille qui lui permet de garantir encore suffisamment de stabilité mais d'un encombrement minimal pour libérer l'espace disponible pour des mesures médicales ou autres appareils.

5. Dispositif selon la revendication 3 ou 4, **caractérisé en ce que** le pied (5) présente des roulettes et un dispositif pour bloquer la mobilité des roulettes.

6. Dispositif selon la revendication 2, **caractérisé en ce que** le pied (5) peut être relié à une partie qui présente un lien fixe au patient (29).

7. Dispositif selon la revendication 6, **caractérisé en ce que** le pied (5) peut être relié à un dispositif de fixation (32) .

8. Dispositif selon la revendication 6, **caractérisé en ce que** le pied (5) peut être relié à la table du patient (31).

9. Dispositif selon l'une des revendications 1 à 8, **caractérisé en ce qu'**il est prévu un sixième axe de réglage (11, 12, 13, 14, 15 ou 16) qui est associé également à un dispositif de réglage fin et de blocage (17).

10. Dispositif selon l'une des revendications 1 à 9, **caractérisé en ce que** l'organe le plus en avant (10) est muni d'un support (18) pour le logement de différents dispositifs (4).

11. Dispositif selon la revendication 10 **caractérisé en ce que** le support (18) présente pour le mouvement linéaire d'un dispositif (4) un guide (19) orientable en direction (3) d'une cible (2).

12. Dispositif selon la revendication 11 **caractérisé en ce que** le guidage (19) du support (18) s'étend en formant un angle avec l'organe le plus en avant (10).

13. Dispositif selon l'une des revendications 1 à 10, **caractérisé en ce que** l'organe avant (10) de l'arbre (7) pour le mouvement linéaire d'un dispositif (4) un guide (20) orientable en direction (3) d'une cible (2).

14. Dispositif selon l'une des revendications 1 à 10, **caractérisé en ce que en ce que** pour le déplacement linéaire d'un dispositif (4), il est prévu un autre axe de réglage (21) qui présente un accouplement a ces deux axes de réglage (14, 16) dont les organes (9, 10) sont mobiles dans les mêmes plans parallèles de sorte que les axes de réglage (21, 14, 16) exécutent des positions angulaires opposées de manière opposée qui s'annulent et peuvent en même temps effectuer une correction pour le maintien de la position du dispositif (4) en direction (3) d'une cible (2) .

15. Dispositif selon l'une des revendications 1 à 14, **caractérisé en ce que** les axes de réglage (11, 12, 13, 14, 15, 16 et le cas échéant 21) sont munis de rapporteurs d'angle (22).

16. Dispositif selon l'une des revendications 1 à 15 **caractérisé en ce qu'**il est reliable à un ordinateur (26) et présente un programme pour un ordinateur (26) qui met en relation, par les positions angulaires des axes de réglage (11, 12, 13, 14, 15, 16 et le cas échéant 21), une cible librement sélectionnable (2) et un emplacement librement sélectionnable du pied (5, 6) de sorte que l'un d'entre eux peut être calculé sur la base de coordonnées connues.

17. Dispositif selon la revendication 16, **caractérisé en ce qu'**il est prévu un dispositif pour la détermination des coordonnés de n'importe quel emplacement du pied (5, 6).

18. Dispositif selon la revendication 16 en association avec la revendication 17, **caractérisé en ce qu'**il présente un programme pour un ordinateur (26) qui calcule pour des emplacement du pieds (5, 6) les positions angulaires des axes de réglage (11, 12, 13, 14, 15, 16 et le cas échéant 21) pour atteindre une cible (2).

19. Dispositif selon la revendication 16 en association avec la revendication 17, **caractérisé en ce qu'**il présente un programme pour un ordinateur (26) qui calcule sur la base de l'emplacement du pied (5, 6) et des positions angulaires des axes de réglage (11, 12, 13, 14, 15, 16 et le cas échéant 21) la position d'une cible (2) abordée par un dispositif (4).

20. Dispositif selon la revendication 17, 18 ou 19, **caractérisé en ce que** le bras (7) est muni d'un dispositif de détermination de position.

21. Dispositif selon l'une des revendications 17 à 20, **caractérisé en ce qu'**il présente un programme pour un ordinateur (26) qui définit la position d'emplacements quelconques choisis du pied (5, 6) au moyen de trois points de calibrage (24, 24', 24") définis comme points cibles (2) .

22. Dispositif selon la revendication 21, **caractérisé en ce que** les points de calibrage (24, 24', 24"9 présente un lien défini au patient (29) et de ce fait la position relative du pied (5,6 ) par rapport au patient (29) peut être déterminée.

23. Dispositif selon la revendication 21 ou 22, **caractérisé en ce que** le programme détermine, après la détermination de l'emplacement du pied (5, 6) au moyen des points de calibrage (24, 24', 24"), les positions angulaires des axes de réglage (11, 12, 13, 14, 15, 16 et le cas échéant 21) pour atteindre une cible prédéterminée (2).

24. Dispositif selon la revendication 21 ou 22, **caractérisé en ce que** le programme vérifie après la détermination de l'emplacement du pied (5, 6) au moyen des points de calibrage (24, 24', 24") à partir des positions angulaires des axes de réglage (11, 12, 13, 14, 15, 16 et le cas échéant 21) la position d'au moins une cible abordée (2).

25. Dispositif selon la revendication 24, **caractérisé en ce que** la position de plusieurs marquages (36) servant de cibles (2) sur le patient (29) ou de marquages (36, 24, 24' 24") avec un lien direct avec le patient (29) servent à déterminer et/ou à vérifier leur position.

26. Dispositif selon l'une des revendications 18 à 23, **caractérisé en ce que** le programme détermine également la course d'insertion (25) jusqu'à atteindre une cible (2) se trouvant dans le corps d'un patient (29).

27. Dispositif selon la revendication 16 á 26, **caractérisé en ce qu'**il est muni d'un ordinateur (26) qui est chargé d'un programme pour la détermination de la position d'au moins une cible (2) par rapport à l'emplacement du pied (5, 6) ou inversement.

28. Dispositif selon l'une des revendications 17 à 27, **caractérisé en ce que** le rapporteur d'angle (22) et le dispositif de détermination de position sont reliés à l'ordinateur (26) lequel détermine les données de localisation du pied (5, 6) les paramètres de la mécanique (1) et les données d'une cible (2).

29. Dispositif selon la revendication 28, **caractérisé en ce que** la détermination des données sert à atteindre une cible prédéterminée (2).

30. Dispositif selon la revendication 28, **caractérisé en ce que** la détermination des données sert à atteindre une cible abordée (2).

31. Dispositif selon l'une des revendications 17 à 30, **caractérisé en ce que** le programme est réalisé de sorte qu'un ordinateur (26) affiche les données déterminées sur un dispositif d'affichage (27, 27').

32. Dispositif selon la revendication 29 ou 31, **caractérisé en ce qu'**également les résultats des réglages effectués sont saisis par le rapporteur d'angle (22) et affichés par le dispositif d'affichage (27, 27').

33. Dispositif selon l'une des revendications 1 à 32, **caractérisé en ce qu'**il est muni d'un système de navigation pour la saisie de position d'une cible abordée (2) par la sonde (4), qui est affichée sur un respectivement sur le dispositif d'affichage (27, 27').

34. Dispositif selon la revendication 33, **caractérisé en ce qu'**il est muni d'un système de caméra ou de palpeur pour la saisie d'une cible (2) abordée par le dispositif (4).

35. Dispositif selon l'un des revendications 1 à 34, **caractérisé en ce qu'**il est relié à un système de localisation stéréotaxique qui saisit la position du dispositif (4) par rapport à la cible (2).

36. Dispositif selon la revendication 35, **caractérisé en ce que** la position du dispositif (4) par rapport à la cible (2) est affichée sur un écran (27').

37. Dispositif selon l'une des revendications 16 à 36, **caractérisé en ce que** les axes de réglages (11, 12, 13, 15, 16) sont munis d'entraînement et **en ce qu'**un ordinateur (26) peut être paramétré au moyen d'un programme pour le réglage automatique des positions angulaires nécessaires.

38. Dispositif selon la revendication 37, **caractérisé en ce qu'**au moyen d'un autre entraînement, le dispositif (4) peut être guidé sur la course d'insertion (25) à la cible (2).

39. Dispositif selon la revendication 38, **caractérisé en ce que** le programme est réalisé de sorte qu'il amène au moyen de l'ordinateur (26) les entraînements et éventuellement un autre entraînement associé au dispositif (4) à effectuer une intervention diagnostique ou thérapeutique.

40. Dispositif selon la revendication 39, **caractérisé en ce que** l'ordinateur (26) est muni d'un dispositif de saisie (28) pour la commande de l'intervention.

41. Dispositif selon l'une des revendications 16 à 40, **caractérisé en ce que** les dispositifs de réglage fin de blocage (17) stoppent automatiquement les axes de réglage (11 à 16) quand la position de consigne respective est atteinte.

42. Dispositif selon l'une des revendications 16 à 41, **caractérisé en ce qu'**il est muni d'un dispositif pour l'émission d'un signal d'avertissement quand l'un des réglages de la mécanique (1) n'est plus dans sa position de consigne.

43. Dispositif selon l'une des revendications 16 à 42, **caractérisé en ce qu'**au moins les zones avant du bras (7) sont fabriquées en matériaux électriquement non conducteurs .et non magnétisables.

44. Dispositif selon l'une des revendications 1 à 43, **caractérisé en ce que** la longueur du bras (7) est dimensionnée pour atteindre des cibles (2) sur l'ensemble du corps humain (29).

45. Dispositif selon l'une des revendications 1 à 43, **caractérisé en ce que** la longueur du bras (7) est dimensionnée pour atteindre des cibles (2) sur une zone partielle du corps humain (29).

46. Dispositif selon l'une des revendications 1 à 43, **caractérisé en ce que** la longueur du bras (7) est dimensionnée pour atteindre des cibles à la tête (29).

47. Dispositif selon l'une des revendications 1 à 46, **caractérisé en ce qu'**il est associé à un fantôme de mesure qui présente une cible de contrôle qui peut être amenée dans la position d'une cible (2) et **en ce qu'**un dispositif de détermination de position saisit l'arrivée de la cible de contrôle.

48. Dispositif selon la revendication 47, **caractérisé en ce qu'**un palpeur (23) peut être placé comme dispositif (4) et sert de dispositif de détermination de position.

49. Dispositif selon l'une des revendications 1 à 48, **caractérisé en ce que** le pied (5) peut être fixé au plafond au-dessus du patient (29).
